# EUROPÄISCHE PATENTSCHRIFT

(11) **EP 2 621 383 B1**
(45) Veröffentlichungstag und Bekanntmachung des Hinweises auf die Patenterteilung: **20.01.2016**
(21) Anmeldenummer: 11763923.7
(22) Anmeldetag: 28.09.2011
(51) Int. Cl.: A61B 17/70, A61B 17/88

(54) **BILATERALES LAMINA-IMPLANTAT**
BILATERAL LAMINA IMPLANT
IMPLANT BILATÉRAL DE LAME VERTÉBRALE

(30) Priorität: 28.09.2010 EP 10011155
(43) Veröffentlichungstag der Anmeldung: 07.08.2013
(73) Patentinhaber: FACET-LINK Inc., Rockaway NJ 07866 (US)
(72) Erfinder: JENSEN, Harm-Iven, 24214 Noer (DE); LINK, Helmut, D., 22397 Hamburg (DE)
(74) Vertreter: Glawe, Delfs, Moll
(86) Internationale Anmeldenummer: PCT/EP2011/066923
(87) Internationale Veröffentlichungsnummer: WO 2012/041927

(56) Entgegenhaltungen:
- WO-A1-2006/104487
- US-A1- 2010 057 127
- US-A1- 2010 106 190
- US-A1- 2010 152 854

## Beschreibung

Die Erfindung betrifft ein bilaterales Verstärkungsimplantat für die Lamina eines Wirbels, umfassend einen Querträger und je eine Verankerungsvorrichtung für die linke und rechte Seite der Lamina des Wirbels.

Die Wirbelsäule bildet ein zentrales Strukturelement des menschlichen Skeletts. Sie umfasst eine Vielzahl von Wirbeln, die für die Übertragung von Lasten übereinander angeordnet und zur Ermöglichung von Bewegungen gelenkig miteinander verbunden sind. Die Wirbel der Wirbelsäule sind nicht identisch, sondern unterscheiden sich in ihrer Gestalt je nach ihrer Anordnung in der Wirbelsäule. Sie weisen jedoch einige Gemeinsamkeiten auf. So weist jeder Wirbel einen massiven Wirbelkörper mit zwei seitlich nach hinten vorstehenden knöchernen Vorsprüngen (Pedikel) auf, die wiederum in ihrem hinteren Teil über einen knöchernen Bogen verbunden sind. Im Verbindungsbereich ist dieser knöcherne Bogen als breite Platte (Lamina) ausgebildet, und weist in seiner Mitte einen nach hinten abragenden spinalen Vorsprung auf. Der spinale Vorsprung (pozessus spinalis) wie auch zwei weitere transverse Vorsprünge an den Seitenflächen der Pedikel bilden Anlenkpunkte für Muskeln und Bänder. In dem Bereich, in dem die Pedikel in die breite Lamina übergehen, sind auf jeder Seite des Wirbels ein oberer und ein unterer Gelenkvorsprung angeordnet. Sie bilden jeweils Teil eines Facettengelenks mit einem benachbarten oberen bzw. unteren Wirbel. Weiter ist zur Lastübertragung der Wirbel vorgesehen, dass zwischen den Wirbelkörpern benachbarter Wirbel jeweils eine Bandscheibe angeordnet ist, die den Zwischenraum zwischen den verhältnismäßig ebenen Deckflächen benachbarter Wirbelkörper ausfüllt. Der von der Rückseite des Wirbelkörpers und dem knöchernen Bogen (Wirbelbogen) umgrenzte Bereich bildet einen Hohlraum, in welchem parallel zur Wirbelsäule laufende Nervenstränge aufgenommen sind.

Erkrankungen oder Verletzungen können zu vielfältigen Arten von Rückenschmerzen führen. Häufige Ursachen hierfür sind insbesondere Defekte an der Bandscheibe, an den Facettengelenken oder Einklemmungen bzw. Einschnürungen der in dem Hohlraum verlaufenden Nervenstränge. Insbesondere im Hinblick auf den letzteren Fall ist es bekannt, dass dieser Druck häufig erzeugt wird von im Bereich des Hohlraums sich bildenden knöchernen Zuwachsungen. Um den Schmerz zu bekämpfen, muss der Druck vermindert und dazu die Zuwachsung entfernt werden. Üblicherweise wird hierzu durch die Rückseite des Wirbelbogens, in der Regel also durch die Lamina, ein Zugang zu dem Hohlraum geschaffen, um dort mittels geeigneter und an sich bekannter Instrumente die störenden Zuwachsungen zu entfernen. Je nachdem ob der Zugang nur auf einer Seite des Wirbels erfolgt, also in einem Bereich der Lamina seitlich des spinalen Vorsprungs, spricht man von einem unilateralen Zugang oder, wenn er auf beiden Seiten des spinalen Vorsprungs erfolgt und dieser in der Folge entfernt wird, von einem bilateralen Zugang. Bei dieser sogenannten Laminektomie wird herkömmlicherweise die in der Lamina geschaffene Öffnung nach der Operation meist nicht wieder verschlossen. Es hat sich gezeigt, dass dies im Hinblick auf die mechanische Stabilität sowie im Hinblick auf die Komplikationsrate über einen längeren Zeitraum hinweg problematisch sein kann.

Aus US 2010/0152854 A1 ist bekannt, ein expandierbares Implantat für die Lamina eines Wirbels vorzusehen. Das expandierbare Implantat umfasst ein Expansionselement mit einem männlichen Stück, das in einem weiblichen Stück längsverschieblich gelagert ist. Weiter weist das Implantat an den Enden des männlichen und weiblichen Stücks jeweils ein Backenelement mit voneinander weg weisenden Kontaktflächen für die Lamina auf. Ein in das weibliche Element eingebrachtes aushärtendes Material verhindert, dass das männliche Stück von der Lamina in das weibliche Stück geschoben wird.

Die Erfindung hat sich zur Aufgabe gesetzt, die mechanische Stabilität des Wirbels wieder herzustellen und somit die Folgeprobleme zu verringern bzw. zu beseitigen.

Die erfindungsgemäße Lösung liegt in den Merkmalen des unabhängigen Anspruchs. Vorteilhafte Weiterbildungen der Erfindung sind Gegenstand der abhängigen Ansprüche.

Bei einem bilateralen Verstärkungsimplantat für die Lamina eines Wirbels umfassend einen Querträger und je eine Verankerungsvorrichtung für die linke und rechte Seite der Lamina des Wirbels wobei am Querträger ein Expansionselement mit einer Führeinrichtung und Backenelementen vorgesehen ist, die entlang der Führeinrichtung längsverschieblich gelagert sind, wobei an voneinander weg weisenden Außenseiten der Backenelemente Anlageflächen für die Lamina ausgebildet sind, ist gemäß der Erfindung mindestens eines der Backenelemente mit einer Ausrichteinrichtung zur veränderbaren Orientierung seiner Anlagefläche relativ zum Expansionselement versehen.

Kern der Erfindung ist der Gedanke, durch das auf die Backenelemente wirkende Expansionselement eine sichere Verankerung des Verstärkungsimplantats an den bei der Laminektomie entstandenen Schnittflächen zu erreichen. Dies kann unter elastischer Aufweitung der Lamina bzw. des Wirbelbogens geschehen, um so die Befestigungssicherheit weiter zu erhöhen. Weiter hat dies in Kombination mit den außen gelegenen Anlageflächen der Backenelemente den Vorteil, dass ein Kollabieren des Wirbelbogens, wie es bisher in unerwünschter Weise geschehen konnte, unmöglich gemacht ist. Im Gegenteil, unter dem bisher zum Kollabieren führenden Druck wird das Verstärkungsimplantat nur fester in seinen Sitz gepresst und kann damit seine Aufgabe erfüllen. Obgleich die elastische Aufweitung einen langzeitstabilen Sitz an sich gewährleisten kann, kann zusätzlich eine Rücklaufsperre für die Backenelemente vorgesehen sein, um so die langfristige Befestigungssicherheit weiter zu erhöhen.

Vorzugsweise ist das zweite Backenelement ebenfalls mit einer Ausrichteinrichtung versehen. Damit kann auch das zweite Backenelement in Bezug auf die Orientierung seiner Außenfläche zu der Führeinrichtung verändert werden. Dies ermöglicht eine feinere Anpassung des Verstärkungsimplantats an die tatsächlichen anatomischen Verhältnisse nach der Laminektomie. Insbesondere ermöglicht es, die Anlagenflächen der Backenelemente keilförmig zueinander auszurichten, und dabei dennoch das Implantat insgesamt verkippungsfrei zu befestigen.

Die Rücklaufsperre ist mit Vorteil als eine zwischen den Backenelementen und der Führeinrichtung wirkende Klemmeinrichtung ausgebildet. Mit einer solchen Klemmung lässt sich nach dem Expandieren auf einfache Weise die erreichte Expansionsposition fixieren. Damit wird ein Verrutschen der Backenelemente verhindert. Sollten die Anforderungen an die Sicherheit gegenüber unerwünschten Bewegungen der Backenelemente höher sein, so kann vorzugsweise die Rücklaufsperre mit Verrastungselementen versehen sein, die zwischen den Backenelementen und der Führeinrichtung angeordnet sind. Zweckmäßigerweise umfassen die Verrastungsmittel eine Riffelung und in sie eingreifende Rastnasen. Mit einer solchen Verrastung, wie sie durch die Riffelung in Kombination mit der Rastnase erreicht ist, ergibt sich eine formschlüssige Sicherung. Dies bietet den Vorteil, auch bei sehr aktiven Patienten mit entsprechender Belastung der Wirbelsäule eine ausreichend sichere Halterung des Verstärkungsimplantats zu erreichen.

Es hat sich bewährt, wenn das Expansionselement mindestens einen Bereich aufweist, der die Backenelemente seitlich überragt. Damit ist unabhängig von der tatsächlich eingestellten Expansionsposition sichergestellt, dass das Verstärkungsimplantat mit seinen Backenelementen nur so weit in die durch die Laminektomie geschaffene Öffnung eingeschoben werden kann, bis die zu den Backenelementen weisende Anlagefläche der Führeinrichtung auf der Lamina anliegt. Dies verhindert ein zu weites Einschieben des Verstärkungsimplantats mit unerwünschten Folgen im Hinblick auf das Einbringen von Druckschmerz auf die im Hohlraum verlaufenden Nerven oder gar das Hervorrufen von Beschädigungen bzw. Verletzungen dieser Nerven.

Die Führeinrichtung ist vorzugsweise als eine unrunde Stange ausgeführt. Unter "unrund" wird hierbei verstanden, dass sie im Querschnitt nicht kreisförmig ist. Dank dieser Unrundheit sind die an der Führungsstange geführten Backenelemente gegenüber einer unerwünschten Verdrehung durch Formschluss gehindert. Mit Vorteil ist die Stange V-förmig, wobei sie vorzugsweise in der Mitte einen V-Winkel von 10° bis 20° einschließt. Besonders bewährt hat sich ein V-Winkel von 20°. Damit passt sich die Führungsstange auf besonders günstige Weise in die Anatomie des rückseitigen Wirbels ein. Insbesondere trägt sie damit kaum nach außen auf, so dass Irritationen umliegenden Gewebes auf ein Minimum reduziert sind.

Es kann auch vorgesehen sein, dass die Führeinrichtung eine gabelförmige Schiene und einen im Gabelzwischenraum geführten Gleiter umfasst. Damit ist die Führung umschlossen sowie vor Beeinträchtigungen geschützt und Irritationen des umliegenden Gewebes werden vermieden. Mit Vorteil ist der Gleiter in der Weise an der Schiene geführt, dass die Seitenleisten formschlüssig in ein Nutenpaar greifen. Meist wird es so sein, dass an der gabelförmigen Schiene ein Paar einander zuweisender Nuten angeordnet sind, während an gegenüberliegenden Lateralseiten des Gleiters entsprechende komplementäre Seitenleisten angeordnet sind, welche formschlüssig in die Nuten eingreifen und den Gleiter längsverschieblich in ihnen führen. Es ist aber auch die umgekehrte Anordnung denkbar.

Grundsätzlich kann der Gleiter durch eine von außen aufgebrachte Bewegung verschoben werden. Es kann aber auch vorgesehen sein, dass eine Antriebseinrichtung für den Gleiter vorgesehen ist, die sich auf der Schiene abstützt. Hierbei kann es sich insbesondere um eine Schraubenspindel oder um eine Zahnstange handeln. Für letzteres ist eine zum Gleiter weisende Kante der Schiene als Zahnstange ausgebildet, während am Gleiter die Anordnung eines mit der Zahnstange kämmenden Ritzels vorgesehen ist. Durch Verdrehen des Ritzels bewegt sich der Gleiter dann längs der Schiene. Es ist hierbei nicht unbedingt erforderlich, dass das Ritzel dauerhaft an dem Gleiter angeordnet ist, sondern es kann genügen, wenn es temporär zur Verstellung an dem Gleiter vorgesehen ist. An dem Gleiter ist vorzugsweise eine Lagerbohrung für das Ritzel ausgebildet. Die praktische Handhabung kann weiter vereinfacht sein, wenn die Antriebseinrichtung selbsthemmend ausgeführt ist. Darunter wird verstanden, dass sie sich ohne Aufbringung einer Verstellkraft nicht selbsttätig unter dem Einfluss einer auf den Gleiter einwirkenden Kraft bewegt; insbesondere ist hiermit gemeint, dass sich bei nicht betätigter Antriebseinrichtung das Expansionselement nicht zusammendrücken lässt, sondern eine solche Bewegung durch die Selbsthemmung blockiert ist. Damit kann die bei der Verstellung erreichte Position noch zusätzlich zu einer Rücklaufsperre, insbesondere einer Klemmeinrichtung, gesichert sein.

Die Anlageflächen an den Backenelementen weisen vorzugsweise spitze Hervorstehungen (Spikes) auf. Bewährte Formen für solche Spikes sind bspw. Kegelspitzen, Pyramiden, prismatische oder V-förmige Erhebungen. Damit kann eine sichere primäre Fixation erreicht werden. Um zusätzlich auch eine schnelle und sichere sekundäre Fixation zu erreichen, sind vorzugsweise die Anlageflächen mit einer knochenwachstumsfördernden Beschichtung versehen. Hierbei kann es sich insbesondere um Hydroxylapatit oder andere osteoinduktive Substanzen handeln.

Die Anlageflächen sind vorzugsweise so an den beiden Backenelementen angeordnet, dass sie miteinander fluchten. Hierunter wird verstanden, dass sie in Richtung des Verstellwegs des Expansionselements gesehen weder einen horizontalen noch vertikalen Offset aufweisen. Damit wird eine asymmetrische Kraftbeaufschlagung des Verstärkungsimplantats verhindert, so dass keine unerwünschten Drehmomente auf das Verstärkungsimplantat wirken, welche es aus seiner vorgesehenen Position zu drehen trachten.

Mit Vorteil ist an den Backenelementen mindestens eine Fixierzunge angeordnet. Sie ist zweckmäßigerweise so ausgebildet, dass sie im implantierten Zustand am Wirbel an einer Außenfläche des Pars aufliegt. Vorzugsweise ist der Winkel der Fixierzunge in Bezug auf das Backenelement veränderbar, um in Abhängigkeit von der individuellen Anatomie eine gute Anlage zu erreichen. In praktisch günstiger Weise kann dies realisiert sein durch eine biegsame Ausbildung der Fixierzunge, vorzugsweise mit einer Materialverdünnung im Bereich des Übergangs zwischen Fixierzunge und Backenelement.

Mit Vorteil weist die Fixierzunge eine Aufnahmeöffnung für eine Befestigungseinrichtung auf. Die Aufnahmeöffnung ist zweckmäßigerweise zur polyaxialen Lagerung einer Pars-Schraube ausgebildet. Unter polyaxial wird verstanden, dass die Schraube mit ihrem Kopf eine sichere flächige Auflage im Bereich der Aufnahmeöffnung nicht nur in einer zentrischen Lage hat, sondern auch bei Winkelabweichungen von bis zu 15° in jede Richtung. Damit kann auch bei unterschiedlicher Anatomie des Wirbels die Pars-Schraube stets in einer für die Befestigung günstigen Orientierung eingesetzt sein. Die Befestigungssicherheit verbessert sich dadurch. Vorzugsweise ist die Aufnahmeöffnung länglich und weist mehrere definierte Aufnahmepositionen für die Befestigungseinrichtung auf. Die definierten Aufnahmepositionen ermöglichen es, unterschiedliche Positionen für die Befestigungseinrichtung (insbesondere eine Schraube) in Relation zu der Fixierzunge vorzusehen. Zweckmäßigerweise sind dazu mehrere Nasen zur Unterteilung vorgesehen, so dass in jeder Aufnahmeposition eine Pars-Schraube - anders als bei einem reinen Langloch - formschlüssig gehaltert ist. Insgesamt ist es so ermöglicht, die Pars-Schraube nicht nur mit einem translatorischen, sondern auch mit zwei rotatorischen Freiheitsgraden im Verhältnis zur Fixierzunge anzuordnen, was auch bei schwieriger Anatomie eine sichere Befestigung erlaubt. An dem Expansionselement sind zweckmäßigerweise Aufnahmekupplungen für ein Spreizinstrument vorgesehen. Hierbei kann es sich insbesondere um als Sackbohrung ausgeführte Passlöcher handeln. Über sie kann das Spreizinstrument schnell und einfach an das Expansionselement angekoppelt sein. Vorzugsweise sind dazu Spreizarme des Spreizinstruments mit zur Aufnahmekupplung komplementären Mitnehmern versehen. Damit ist eine Art Schnellkupplung gebildet, welche auf besonders einfache und komfortable Weise eine schnelle Verbindung zwischen Spreizinstrument und Implantat erlaubt.

Zweckmäßigerweise ist das Spreizinstrument als eine Spreizzange ausgebildet, die einen Schaft und einen Handgriff umfasst. Mit einer Spreizzange kann auf bequeme und für den Operateur nahezu transparente Weise eine Betätigung des Expansionselements erreicht sein. Zweckmäßigerweise ist eine Übertragungsmechanik zwischen Schaft und Handgriff vorgesehen, welche eine im Handgriff eingeleitete Betätigungsbewegung in ihrem Weg vergrößert. Dazu ist zweckmäßigerweise die Übertragungsmechanik mit einem doppelt wirkenden L-förmigen Hebel versehen, dessen Drehpunkt den Hebel im Verhältnis von mindestens 2:1 teilt.

Es hat sich bewährt, wenn die Übertragungsmechanik im Schaft angeordnet ist. Eine solche Anordnung der Übertragungsmechanik ist raumsparend und ermöglicht so die Verwendung auch unter beengten Platzverhältnissen ohne Beeinträchtigung des umliegenden Gewebes. Mit Vorteil ist die Übertragungsmechanik so ausgebildet, dass die Spreizer eine Linearbewegung ausführen. Damit wird im Vergleich zu einer herkömmlichen - rotatorischen - Spreizbewegung eine exaktere Führung der Elemente des Expansionselements, insbesondere des Gleiters, erreicht. Der Gefahr von Verklemmungen, wie sie bei kreisbogenförmig geführten Spreizern auftreten würden, kann auf diese Weise entgegengewirkt werden.

Vorzugsweise ist ein Antriebsinstrument vorgesehen, welches einen Schaft mit einem Kupplungskopf an einem Ende und einem Handgriff am anderen Ende sowie eine zur Seite wegweisende Winkelmarkierung aufweist. Mit einem solchen Antriebsinstrument ist das Verstärkungsimplantat auch an seinem vorgesehenen Implantationsort leicht und sicher zu betätigen und in eine gewünschte Spannposition zu verbringen. Insbesondere ist es mit der Winkelmarkierung ermöglicht, eine Antriebseinrichtung (bspw. mit einer Zahnstange) um eine definierte Strecke zu betätigen, indem einfach eine bestimmte Winkeldifferenz beim Betätigen des Antriebsinstruments erfolgt. Damit erhöht sich die Reproduzierbarkeit des Spreizvorgangs und zum anderen bietet dies auch weniger erfahrenen Operateuren die Möglichkeit, den Expansionsweg reproduzierbar zu bestimmen. Mit Vorteil sind Winkelmarkierung und Handgriff miteinander kombiniert. Dies ermöglicht eine besonders raumsparende Konstruktion des Antriebsinstruments.

Die Erfindung wird nachfolgend unter Bezugnahme auf die beigefügte Zeichnung anhand eines Ausführungsbeispiels näher erläutert. Darin zeigen:
- Fig. 1 a, b:: eine perspektivische Ansicht eines ersten Ausführungsbeispiels im demontierten und montierten Zustand;
- Fig. 2 a, b:: eine perspektivische Ansicht eines zweiten Ausführungsbeispiels im demontierten und montierten Zustand;
- Fig. 3 a, b:: eine perspektivische Ansicht eines dritten Ausführungsbeispiels im demontierten und montierten Zustand;
- Fig. 4 a-d:: eine perspektivische Ansicht eines vierten Ausführungsbeispiels im demontierten und montierten Zustand;
- Fig. 5:: eine Explosionsansicht eines Spannwerkzeugs für das Verstärkungsimplantat;
- Fig. 6 a-c:: verschiedene Stufen des Spannens des Implantats;
- Fig. 7 a, b:: ein Antrieb für ein Verstärkungsimplantat; und
- Fig. 8 a-c:: eine Ansicht von Wirbeln mit dem Verstärkungsimplantat.

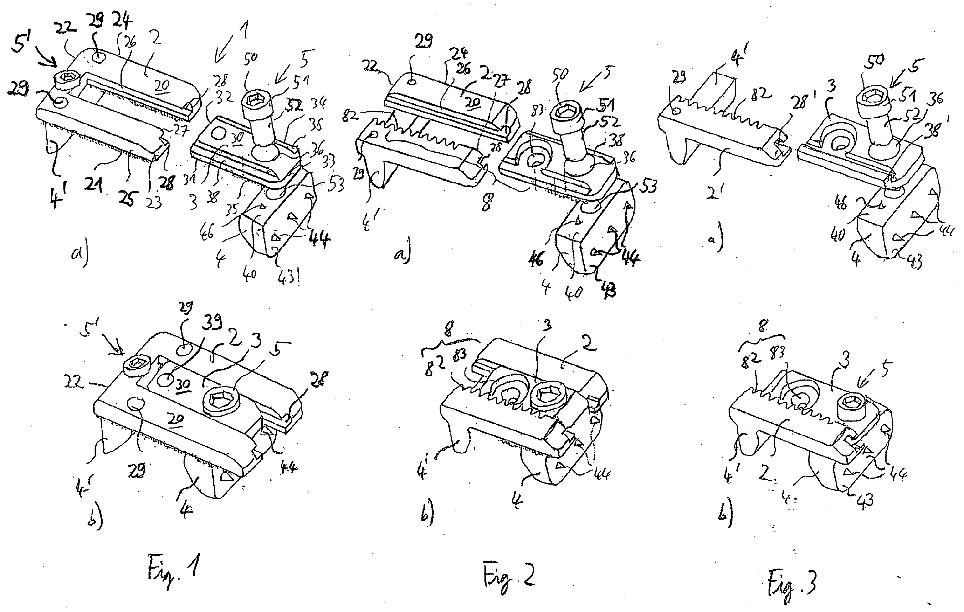

Bei dem in Fig. 1 dargestellten ersten Ausführungsbeispiel für ein erfindungsgemäßes Verstärkungsimplantat handelt es sich um eine so genannte Gabelvariante. Das Verstärkungsimplantat weist als Führeinrichtung einen gabelförmigen Kulissenkörper 2 auf, in dem ein Schlitten 3 längsverschieblich geführt ist. An dem Kulissenkörper 2 sowie an dem Schlitten 3 ist jeweils ein Backenelement 4, 4' angeordnet. Sie weisen an ihren jeweils nach außen, d. h. voneinander wegweisenden Seiten 43, eine Anlagefläche für eine Lamina des Wirbels auf.

Der gabelförmige Kulissenkörper 2 und der Schlitten 3 wirken derart zusammen, dass der Schlitten 3 längsverschieblich an dem Kulissenkörper 2 geführt ist. Damit ist ein Expansionselement 1 gebildet, welches den Abstand zwischen den beiden Außenflächen 43 der beiden Backenelemente 4,4' verschieden groß einstellen kann. Diese Einstellbarkeit ermöglicht es, mittels des Expansionselements durch Bewegen des Schlittens 3 längs seiner Führung an dem gabelförmigen Kulissenkörper 2 ein Verstärkungsimplantat zu schaffen, welches unterschiedlich breite durch Resektion entstandene Zwischenräume in der Lamina eines Wirbelkörpers überbrücken kann.

Der gabelförmige Kulissenkörper 2 weist eine Oberseite 20, eine Unterseite 21, mit einer Anschrägung versehene Stirnseiten 22, 23 sowie im wesentlichen plane Lateralseiten 24, 25 auf. Parallel zu den Lateralseiten 24, 25 sind im Gabelzwischenraum Führungsflächen 26, 27 ausgebildet, längs derer der Schlitten 3 verschieblich ist. Zur präziseren und verliersicheren Führung ist in den Führungsflächen 26, 27 jeweils eine parallel zur Unterseite 21 verlaufende Führungsnut 28 ausgebildet. Sie ist offen zu der Stirnseite 23 und mündet im Übergangsbereich zu deren Abschrägung.

Der in seiner Grundform plattenförmige Schlitten 3 weist zwei Stirnseiten 32, 33 sowie zwei Lateralseiten 34, 35 auf. An den beiden Lateralseiten 34, 35 ist jeweils eine Längsleiste 38 angeordnet, die eine zu den Führungsnuten 28 komplementäre Form aufweist und so bemessen ist, dass sie formschlüssig mit minimalem Spiel darin aufgenommen ist. Weiter ist an dem Schlitten 3 nahe der Stirnseite 33 eine Durchgangsöffnung 36 angeordnet, in welcher eine Klemmeinrichtung 5 zum Positionieren und Sichern eines der Backenelemente 4 angeordnet ist. Das andere Backenelement 4' ist am anderen Ende des Expansionselements 1 im Bereich der Stirnseite 22 angeordnet und mittels eines gleichartigen Klemmelements 5' gesichert. Bei dem in Fig. 1 dargestellten Ausführungsbeispiel ist das Klemmelement 5' des zugeordneten Backenelements 4' im Bereich einer Brücke des gabelförmigen Kulissenkörpers 2 angeordnet.

Die Backenelemente 4, 4' sind spiegelsymmetrisch zueinander aufgebaut (sie können auch identisch zueinander ausgeführt sein). Sie weisen an ihrer jeweils von dem anderen Backenelement 4', 4 wegweisenden Außenseite 43 eine Mehrzahl von Spikes 44 zur Verankerung in den Schnittflächen der Lamina auf. Die Außenseiten 43 mit den Spikes 44 sind vorzugsweise mit einer knochenwachstumsfördernden Beschichtung, wie Hydroxylapatit versehen. An ihrer zu der Unterseite 21, 31 des gabelförmigen Kulissenkörpers 2 bzw. des Schlittens 3 weisenden Oberseite 40 sind die Backenelemente 4, 4' vorzugsweise mit einem weiteren, im Regelfall von den Abmessungen her kleineren Spike 46 versehen. Dieser ist dazu ausgebildet, mit der Unterseite 21, 31, die vorzugsweise eine Riffelung aufweist, derart zusammenzuwirken, dass sich unter Einwirkung von durch die Klemmeinrichtung 5 aufgebrachter Klemmkraft eine möglichst feste, vorzugsweise formschlüssige, Verbindung zwischen den Backenelementen 4, 4' sowie dem gabelförmigen Kulissenkörper 2 bzw. dem Schlitten 3 ergibt. Die Backenelemente 4, 4' sind mittels der Klemmeinrichtung 5, 5' an ihrer Oberseite 40 und den darauf angeordneten Spike 46 gegen die Riffelung an der Unterseite 21 bzw. 31 gezogen. Dazu weist die Klemmeinrichtung 5, 5' eine in ihrer Gesamtheit mit der Bezugsziffer 50 bezeichnete Schraube auf, welche einen Schraubenkopf 51 und einen Schaft 52 mit einem Außengewinde aufweist. Der Kopf 51 weist einen größeren Durchmesser als die Durchgangsöffnung 36 in dem Schlitten 3 auf, so dass der Schaft 52 durch sie hindurch in ein entsprechendes Gegengewinde 53 in dem Backenelement 4, 4' greifen kann. Durch Festziehen der Schraube 50 wird somit das Backenelement 4, 4' gegen die Unterseite 31 des Schlittens 3 bzw. auf entsprechende Weise gegen die Unterseite 21 des gabelförmigen Kulissenkörpers 2 gezogen. Damit entsteht sowohl eine kraft- wie auch formschlüssige Verbindung.

Das am Schlitten 3 angeordnete Backenelement 4 weist längs seiner Außenfläche 43 eine größere Abmessung auf als der Abstand zwischen den Lateralseiten 34, 35 des Schlittens 3. Damit steht das Backenelement 4 über den Bereich des Schlittens 3 hinaus und wird von der Klemmeinrichtung 5 so nicht nur gegen die Unterseite 31 des Schlittens 3, sondern ebenso gegen die Unterseite 21 des gabelförmigen Kulissenkörpers 2 gezogen. Unter dem Einfluss der Klemmkraft erfolgt damit eine Fixierung der relativen Lage zwischen dem Schlitten 3 und dem gabelförmigen Kulissenkörper 2, also der jeweiligen Stellung des Expansionselements 1. Damit kann nicht nur eine formschlüssige Sicherung des Backenelements 4 gegenüber einer unerwünschten relativen Verdrehung um die Achse der Schraube 50 des Klemmelements 5 gesichert werden, sondern auch ein unerwünschter Rücklauf des Schlittens 3 unter dem Einfluss einer komprimierenden, auf die Backenelemente 4, 4' einwirkenden Kraft durch den formschlüssigen Eingriff der Spikes 46 in die entsprechende Riffelung an der Unterseite 21 des Kulissenkörpers 2 verhindert werden. Damit ergibt sich auf einfache konstruktive Weise zugleich eine Verdreh- wie auch eine Rücklaufsicherung.

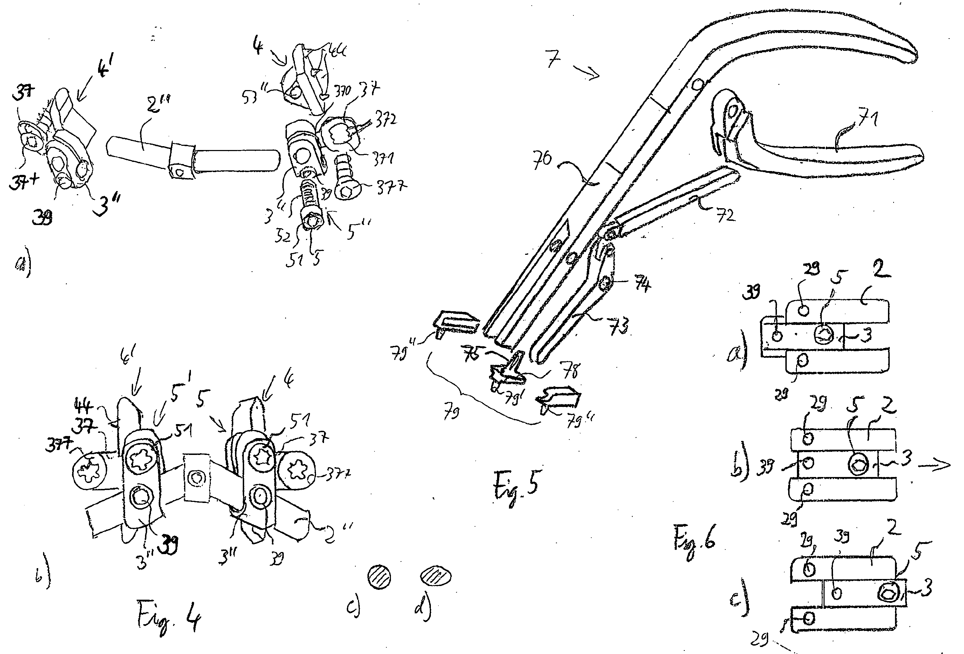

Zum Betätigen des Expansionselements 1 ist ein Spreizinstrument 7 vorgesehen (s. Fig. 5), welches den Schlitten 3 längs der durch die Nuten 28 vorgegebenen Bahn relativ so gegenüber dem gabelförmigen Kulissenkörper 2 verschiebt, dass sich der Abstand zwischen den Backenelementen 4, 4' vergrößert. Um dies von der Oberseite aus bewerkstelligen zu können, sind auf den Oberseiten 20, 30 des gabelförmigen Kulissenkörpers 2 bzw. des Schlittens 3 nahe derer Stirnseiten 22 bzw. 32 mindestens zwei als Passbohrungen ausgeführte Aufnahmeöffnungen 29 bzw. 39 angeordnet. Bei der in Fig. 1 dargestellten Ausführungsform sind zwei Aufnahmeöffnungen 29 auf den beiden die Gabel bildenden Außenelementen des gabelförmigen Kulissenkörpers 2 sowie eine Aufnahmeöffnung 39 an dem Schlitten 3 angeordnet. Die Aufnahmeöffnungen 29 sind als Passlöcher für an einer Spitze des Spreizinstruments 7 angeordnete Mitnehmer ausgeführt. Sie sind als zylinderförmige Dornen 79 ausgeführt, von denen zwei starr in Bezug an einen Schaft 70 des Instruments 7 und einer an einem längsverschieblichen Schubglied 78 des Instruments 7 angeordnet ist. Das Instrument 7 weist an seinem den Dornen 79 gegenüberliegenden Ende des Schafts 70 einen Betätigungsgriff 71 auf, der schwenkbeweglich an dem Schaft 70 gelagert ist. Der Betätigungsgriff 71 wirkt über einen ersten Lenker 72 auf einen L-förmig ausgebildeten Schwenkhebel 73, dessen griffseitiges Ende über ein Schwenklager mit dem Lenker 72 verbunden ist und dessen vorderes Ende über ein Gleitschienenlager 75 auf das Schubglied 78 derart wirkt, dass dieses linear bewegt ist, und zwar so, dass es quer zur Achse der Dornen 79 beweglich ist. Dadurch wird eine Schwenkbewegung des Betätigungsgriffs 71 in eine Linearbewegung des Schubglieds 78 umgesetzt, bei der sich der an dem Schubglied 78 angeordnete Dorn 79' von den fest am Schaft 70 angeordneten Dornen 79" entfernt. Hierbei ist die Anordnung des Dorns 79' relativ zu den Dornen 79" so gewählt, dass in einer Grundstellung (ohne auf den Betätigungsgriff 71 wirkende Handkraft) die Dornen 79', 79" alle in einer Linie sind (s. Fig. 6b), und sich bei Bewegen des Betätigungsgriffs 71 der mittig zwischen den beiden schaftfesten Dornen 79" angeordnete bewegliche Dorn 79' sich aus der Linie heraus entfernt.

Indem die Dornen 79" in die entsprechenden als Passbohrungen ausgeführten Aufnahmeöffnungen 29 an dem gabelförmigen Kulissenkörper 2 und der Dorn 79' in die Aufnahmeöffnung 39 an dem Schlitten 3 eingreift, wird ausgehend von der Grundstellung mit zunehmender Bewegung des Betätigungsgriffs 71 der Schlitten 3 relativ wegbewegt von dem gabelförmigen Kulissenkörper 2 (s. Fig. 6b), wodurch sich der Abstand zwischen den Backenelementen 4, 4' vergrößert und das Expansionselement 1 somit gespreizt wird. Durch Betätigen der Klemmeinrichtung 5 wird die gespreizte Position (s. Fig. 6c) fixiert und gegenüber einem Rücklauf gesichert, so dass das Betätigungsinstrument 7 abgenommen werden kann.

Die formschlüssige Koppelung mittels Dornen 79 in die Öffnungen 29, 39 fungiert nicht nur als Schnellkupplung, die eine schnelle und sichere Einkoppelung bzw. Abtrennung auch ohne visuelle Kontrolle ermöglicht, sondern erlaubt durch den formschlüssigen Eingriff auch eine positive Kraftrückmeldung in der Weise, dass der Operateur am Betätigungsgriff 71 unmittelbar die durch die Spreizung der Backenelemente 4, 4' hervorgerufenen Kräfte spüren kann. Er erhält damit eine feinfühlige Rückkoppelung dafür, welche Anpresskraft der Backenelemente 4, 4' auf die Lamina des Wirbels 9 erreicht ist. Der Operateur kann so die bei der Implantation aufgewendete Kraft entsprechend dosieren.

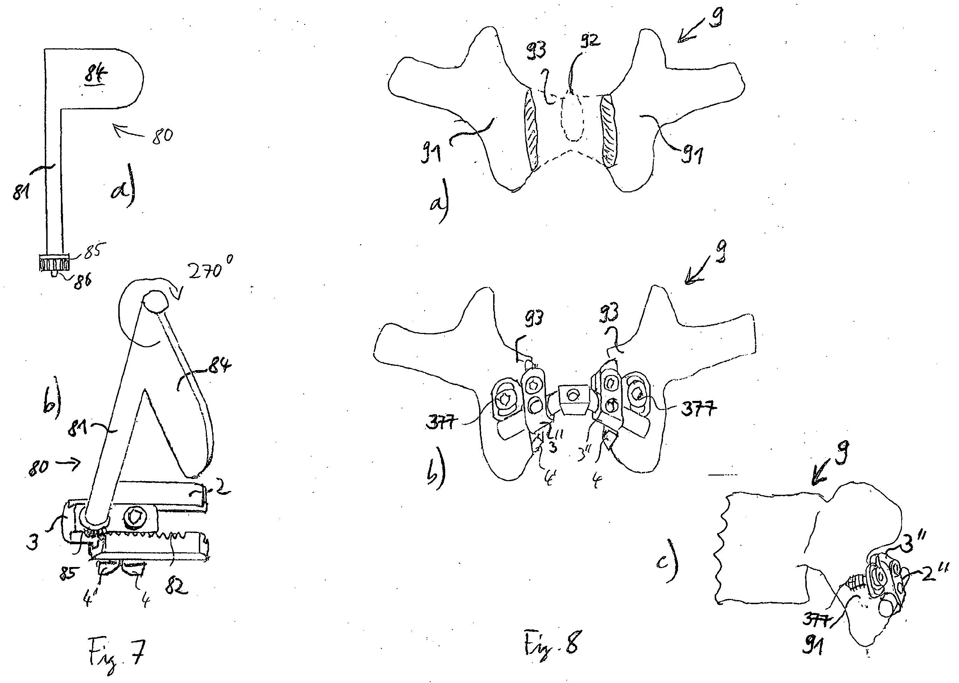

In Fig. 2 ist eine alternative Ausführungsform dargestellt, welche sich von der in Fig. 1 dargestellten Ausführungsform im Wesentlichen dadurch unterscheidet, dass eine integrierte Antriebseinrichtung 8 zum Spreizen des Expansionselements 1 vorgesehen ist. Dazu ist an dem Schlitten 3 eine Aufnahmebohrung 83 als Widerlager für ein Antriebsinstrument 80 vorgesehen, und weiter ist an der Kante zwischen Oberseite 20 und Führungsfläche 27 eine Verzahnung 82 ausgebildet. Sie wirkt als stationärer Teil einer Antriebseinrichtung 8, deren beweglicher Teil gebildet ist durch das in die Aufnahmebohrung 83 eingesetzte Antriebsinstrument 80 (s. Fig. 7a). Es umfasst einen Schaft 81 mit einem Griff 84 an einem einstückig mit dem Schaft ausgeführten Zahnrad 85 am anderen Ende, das mit einem Achsstumpf 86 versehen ist. Das Antriebsinstrument 80 ist zur Betätigung mit seinem Achsstumpf 86 in die Antriebsbohrung 83 des Schlittens 3 gesteckt, wodurch das Zahnrad 85 in Eingriff kommt mit der Verzahnung 82 an dem gabelförmigen Kulissenkörper 2. Die Antriebseinrichtung 8 wird betätigt, indem das Antriebsinstrument 80 an dem Griff 84 ausgehend von einer Grundstellung verdreht wird, wodurch das mit der Verzahnung 82 kämmende Zahnrad 85 sich mit dem Schlitten 3 längs der Verzahnung 82 bewegt und sich das an dem Schlitten 3 angeordnete Backenelement 4 von dem an dem gabelförmigen Kulissenkörper 2 angeordneten Backenelement 4' entfernt. Ist der gewünschte Abstand erreicht, so wird wie bei der in Fig. 1 dargestellten ersten Ausführungsform die Klemmeinrichtung 5 durch Festziehen der Klemmschraube 50 betätigt und der Schlitten so in seiner erreichten Position fixiert. Das Antriebsinstrument 80 kann dann abgenommen werden.

Das Erreichen der gewünschten Position kann hierbei wie bei der ersten Ausführungsform dadurch definiert sein, dass eine bestimmte Verstellkraft erreicht ist. Es gibt aber auch die Möglichkeit, eine bestimmte Verstellstrecke als Kriterium heranzuziehen, die einfach durch einen Betätigungswinkel des Antriebswerkzeugs 8 eingestellt sein kann. Hierbei fungiert der Griff 84 als Winkelindex. Wird das Antriebsinstrument 80 in einer Normallage eingesetzt, und ist ein Verstellweg entsprechend einer Verdrehung von 270° vorgesehen, so wird es durch Verdrehen so weit betätigt, bis der Griff 84 eine dreiviertel Umdrehung entsprechend 270° erreicht. Damit ist die gewünschte Verstellung und damit der gewünschte Abstand zwischen den beiden Backenelementen 4, 4' erreicht. Damit kann eine reproduzierbare Verstellung des Implantats auch unter verschiedenen Einsatzbedingungen erreicht werden.

Es sei angemerkt, dass die in Fig. 2 dargestellte zweite Ausführungsform nicht zwingend mittels der Antriebseinrichtung und dem dazu vorgesehenen Antriebsinstrument 80 verstellt zu werden braucht, sondern dass ebenfalls eine Aufnahmekupplung für ein Spannwerkzeug vorgesehen sein kann, nämlich eben so wie bei der ersten Ausführungsform mit Passbohrungen 29 an dem gabelförmigen Kulissenstück 2' und ein entsprechendes Passloch 39 an dem Schlitten 3'.

In Fig. 3 ist eine dritte Ausführungsform dargestellt, die im Unterschied zu den beiden ersten Ausführungsformen kein gabelförmiges Kulissenstück, sondern einen einseitigen Kulissenkörper 2' aufweist. Die Führungsnut 28' weist einen nach oben gerichteten Hinterschnitt auf, in den ein Hakenfortsatz der Längsleiste 38' greift, und so den Schlitten 3 am einseitigen Kulissenkörper 2' hält. Diese Ausführungsform wird als "Monorail" bezeichnet.

Eine vierte in Fig. 4 dargestellte Ausführungsform unterscheidet sich von den in Fig. 1 bis 3 dargestellten Ausführungsformen im Wesentlichen dadurch, dass sie in Skelettbauweise ausgeführt ist. Das Kulissenstück ist hierbei als eine gerade oder V-förmig abgewinkelte Stange 2" (letztere ist in Fig. 4 dargestellt) ausgeführt. Sie fungiert als Träger für zwei auf sie aufgesteckte Schiebeschlitten 3", die längsverschieblich an der Stange 2" geführt sind. Ferner weist die Stange 2" einen unrunden, in dem dargestellten Ausführungsbeispiel ovalen (s.Fig. 4d), Querschnitt auf und erreicht somit eine Verdrehsicherung der Schiebeschlitten 3" in Bezug auf die Stange 2". Dazu weisen die Schiebeschlitten 3" jeweils eine zur Querschnittsform der Stange 2" kongruente Durchstecköffnung auf. Diese ist Teil einer Klemme, welche durch eine Klemmeinrichtung 5" betätigt ist. Sie umfasst eine Schraube 50, die mit ihrem Kopf 51 auf einer Seite der Klemme gehaltert ist und mit einem an der anderen Seite der Klemme angeordneten Gegengewinde 53" zusammenwirkt. Durch Festziehen der Schraube 50 wird die Klemme betätigt und damit der jeweilige Schiebeschlitten 3" relativ zu der Stange 2" fixiert. Es sei angemerkt, dass der unrunde Querschnitt nicht zwingend ist, sondern dass er auch rund sein kann (s. Fig. 4c).

Zum Betätigen des so gebildeten Expansionselements sind wiederum auf dem Schiebeschlitten 3" Aufnahmekupplungen für das Spannwerkzeug 7 ausgebildet. Dazu weist wie bei den vorgenannten Ausführungsbeispielen der Schiebeschlitten 3" jeweils eine Passbohrung 39 auf, wobei im Unterschied zu den vorstehend genannten Ausführungsformen die Stange 2" keine Passbohrung aufzuweisen braucht. Das Spreizwerkzeug 7 wird mit einer seiner stationären Dornen 79" in eine der Passbohrungen 39 und mit seinem beweglichen Dorn 79' in die andere Passbohrung 39 eingesetzt, wodurch beim Betätigen des Spreizwerkzeugs die beiden Schiebeschlitten 3" voneinander längs der durch die Stange 2" vorgegebenen Führung entfernt werden. Ist die gewünschte Position erreicht, wird sie durch Betätigen der Klemmeinrichtung 5" gesichert, und das Spreizwerkzeug 7 kann abgenommen werden.

Die Backenelemente 4, 4' sind wie bei den vorstehend beschriebenen Ausführungsformen mittels der Klemmeinrichtung gehaltert.

Zur besseren Fixierung des Implantats an dem Wirbel ist eine Fixierzunge 37 vorgesehen, welche nach außen abragend an den Schiebeschlitten 3" angeordnet ist. Es sei angemerkt, dass eine solche Fixierzunge 37 auch bei den übrigen Ausführungsformen vorgesehen sein kann; sie wird nachfolgend in Bezug auf die in Fig. 4 dargestellte Ausführungsform näher erläutert - für die anderen gilt dies sinngemäß entsprechend. Die Fixierzunge 37 ist mit einer Materialverdünnung 370 im Bereich des Übergangs zu dem Schiebeschlitten 3" versehen. Dank dieser Materialverdünnung 370 kann die Fixierzunge 37 in ihrer relativen Winkellage zu dem Schiebeschlitten 3" durch Verbiegen geändert werden. Dies ermöglicht eine Anpassung an die jeweiligen anatomischen Verhältnisse des Wirbels, um so eine optimierte Anlage der Fixierzunge 37 an dem Wirbel, genauer gesagt dessen Pars, zu erreichen. Die Fixierzunge 37 ist an ihrem freien Ende mit einer Befestigungsöffnung 371 versehen. Sie ist vorzugsweise länglich ausgeführt, die auf ihren beiden Längsseiten über jeweils zwei Vorsprünge 372 in drei Bereiche unterteilt ist. Der Rand der Befestigungsöffnung 371 ist schräg geformt, so dass sich zusammen mit den Vorsprüngen 372 eine konische Auflagefläche ergibt. Eine Pars-Schraube 377 mit einem runden Aufnahmekopf kann so an insgesamt drei Positionen in der Befestigungsöffnung 371 gehaltert sein: eine obere Position, eine mittlere Position zwischen den Vorsprungspaaren 372 und eine untere Position. Dabei ist die Pars-Schraube 377 so in eine der Positionen in der Befestigungsöffnung 371 der Fixierzunge 37 gehaltert, dass sie in zwei Richtungen und +/- 15° verschiedene Achsen (polyaxial) annehmen kann. Die Abmessungen der Befestigungsöffnung 371 sind so gewählt, dass die verschiedenen Positionen jeweils 2 mm auseinander liegen, also insgesamt eine Verstellung um 4 mm durch die drei Positionen ermöglicht ist.

Ein Beispiel für die Anordnung des Implantats in einem Wirbel 9 ist in Fig. 8a, b dargestellt. In Fig. 8a ist eine Rückansicht des Wirbels 9 mit einer Lamina 93 dargestellt, wobei durch eine Resektion sowohl ein spinaler Vorsprung 92 sowie angrenzende Bereiche rechts und links davon entfernt sind (durch gestrichelte Linien dargestellt). In die so geschaffene Öffnung in der Lamina 93 wird das Implantat eingesetzt und, wie vorstehend beschrieben, durch Auseinanderbewegen des Expansionselements 1 werden die Backenelemente 4, 4' des Implantats zur Anlage an die bei der Resektion geschaffenen Schnittflächen (schraffiert dargestellt in Fig. 8a) der Lamina 93 gebracht und dagegen verspannt. Das Implantat bildet damit eine die beiden Enden der Lamina 93 verbindende Brücke. Dabei greifen die Spikes 46 an den Außenseiten 43 der Backenelemente 4, 4' in die Schnittflächen an der Lamina 93 ein und sorgen so für eine Primärbefestigung des Implantats. Um das Implantat vor einer Dislokation und gegenüber einer unerwünschten Verdrehung weiter zu schützen, erfolgt beidseitig eine weitere Verankerung durch die Fixierzunge mit der Pars-Schraube 377, und zwar im Pars 91 des Wirbels 9. Letzteres ist zur Verdeutlichung in der teiltransparenten Darstellung in Fig. 8c dargestellt.

## Patentansprüche

1. Verstärkungsimplantat für die Lamina (93) eines Wirbels (9), umfassend einen Querträger und je eine Verankerungseinrichtung für die rechte und linke Seite der Lamina des Wirbels, wobei am Querträger ein Expansionselement (1) mit einer Führeinrichtung (2) und Backenelementen (4, 4') vorgesehen ist, die entlang der Führeinrichtung (2) längsverschieblich gelagert sind, wobei an voneinander weg weisenden Außenseiten der Backenelemente (4, 4') Anlageflächen (43) für die Lamina (93) ausgebildet sind, und eine Rücklaufsperre für die Backenelemente (4, 4') vorgesehen ist,
**dadurch gekennzeichnet, dass**
mindestens eines der Backenelemente (4, 4') mit einer Ausrichteinrichtung zur veränderbaren Orientierung seiner Anlagefläche (43) relativ zur Führeinrichtung (2) versehen ist.

2. Verstärkungsimplantat nach Anspruch 1,
**dadurch gekennzeichnet, dass**
die Rücklaufsperre als eine zwischen Backenelementen (4, 4') und Führeinrichtung (2) wirkende Klemmeinrichtung (5, 5') ausgebildet ist.

3. Verstärkungsimplantat nach Anspruch 2,
**dadurch gekennzeichnet, dass**
die Rücklaufsperre Verrastungselemente aufweist, die zwischen Backenelementen (4, 4') und Führeinrichtung (2) wirken.

4. Verstärkungsimplantat nach Anspruch 3,
**dadurch gekennzeichnet, dass**
die Verrastungselemente eine Riffelung und mindestens eine in sie eingreifende Rastnase umfassen.

5. Verstärkungsimplantat nach einem der Ansprüche 1 bis 4,
**dadurch gekennzeichnet, dass**
eine Klemmschraube (50) vorgesehen ist, die als Lager für die Ausrichteinrichtung fungiert.

6. Verstärkungsimplantat nach einem der Ansprüche 1 bis 5,
**dadurch gekennzeichnet, dass**
das zweite Backenelement (4') mit einer Ausrichteinrichtung (5') versehen ist.

7. Verstärkungsimplantat nach einem der vorangehenden Ansprüche,
**dadurch gekennzeichnet, dass**
das Expansionselement (1) mindestens einen seitlich über die Backenelemente (4, 4') hinausragenden Bereich aufweist.

8. Verstärkungsimplantat nach einem der vorangehenden Ansprüche,
**dadurch gekennzeichnet, dass**
die Führeinrichtung (2) eine unrunde Stange (2") mit einer komplementär geformten Ausnehmung in Schiebeschlitten (3") der Backenelemente (4, 4') umfasst.

9. Verstärkungsimplantat nach Anspruch 8,
**dadurch gekennzeichnet, dass**
die Stange (2") V-förmig ist und vorzugsweise einen V-Winkel von 10° bis 30° bildet.

10. Verstärkungsimplantat nach einem der vorangehenden Ansprüche,
**dadurch gekennzeichnet, dass**
die Führeinrichtung eine gabelförmige Schiene (2) und einen im Gabelzwischenraum geführten Gleiter (3) umfasst.

11. Verstärkungsimplantat nach Anspruch 10,
**dadurch gekennzeichnet, dass**
der Gleiter (3) über Nut und Leiste (28, 38) in der Weise an der Schiene (2) geführt ist, dass die Leiste formschlüssig in die Nut greift.

12. Verstärkungsimplantat nach Anspruch 10 oder 11,
**dadurch gekennzeichnet, dass**
am Gleiter (3) eine Antriebseinrichtung (8) angeordnet ist, die sich auf der Schiene (2) abstützt.

13. Verstärkungsimplantat nach Anspruch 12,
**dadurch gekennzeichnet, dass**
die Antriebseinrichtung (8) eine Zahnstange oder eine ,Schraubenspindel umfasst.

14. Verstärkungsimplantat nach einem der Ansprüche 12 bis 13,
**dadurch gekennzeichnet, dass**
die Antriebseinrichtung (8) selbsthemmend ist.

15. Verstärkungsimplantat nach einem der vorangehenden Ansprüche,
**dadurch gekennzeichnet, dass**
die Anlagefläche (43) spitze Hervorstehungen (46)
aufweist.

16. Verstärkungsimplantat nach einem der vorangehenden Ansprüche,
**dadurch gekennzeichnet, dass**
die Anlagefläche (43) mit einer knochenwachstumsfördernden Beschichtung versehen ist.

17. Verstärkungsimplantat nach einem der vorangehenden Ansprüche,
**dadurch gekennzeichnet, dass**
die Anlageflächen an den Backenelementen (4, 4') miteinander fluchten.

18. Verstärkungsimplantat nach einem der vorangehenden Ansprüche,
**dadurch gekennzeichnet, dass**
an den Backenelementen (4, 4') mindestens eine Fixierzunge (37) angeordnet ist.

19. Verstärkungsimplantat nach Anspruch 18,
**dadurch gekennzeichnet, dass**
die Fixierzunge (37) eine Aufnahmeöffnung (371) für ein Befestigungselement aufweist.

20. Verstärkungsimplantat nach Anspruch 19,
**dadurch gekennzeichnet, dass**
die Aufnahmeöffnung (371) zur polyaxialen Lagerung einer Pars-Schraube (377) ausgebildet ist.

21. Verstärkungsimplantat nach Anspruch 19 oder 20,
**dadurch gekennzeichnet, dass**
die Aufnahmeöffnung (371) länglich und durch mehrere Nasen (372) in eine Mehrzahl von eindeutigen Aufnahmepositionen für das Befestigungselement unterteilt ist.

22. Verstärkungsimplantat nach einem der vorangehenden Ansprüche,
**dadurch gekennzeichnet, dass**
an dem Expansionselement (1) Aufnahmekupplungen für ein Spannwerkzeug (7) vorgesehen sind.

23. Verstärkungsimplantat nach Anspruch 22,
**dadurch gekennzeichnet, dass**
die Aufnahmekupplungen als Passlöcher (29, 39) ausgeführt sind.

24. Verstärkungsimplantat nach Anspruch 23,
**dadurch gekennzeichnet, dass**
ein Spreizinstrument (7) vorgesehen ist, an dessen Spreizarmen zur Aufnahmekupplung komplementäre Mitnehmer (79) vorgesehen sind.

25. Verstärkungsimplantat nach Anspruch 24,
**dadurch gekennzeichnet, dass**
das Spreizinstrument (7) eine Spreizzange ist, die einen Schaft (70) und einen Handgriff (71) umfasst.

26. Verstärkungsimplantat nach Anspruch 25,
**dadurch gekennzeichnet, dass**
eine Übertragungsmechanik vorgesehen ist, welche eine am Handgriff (71) einwirkende Betätigungsbewegung in ihrem Weg vergrößert.

27. Verstärkungsimplantat nach Anspruch 26,
**dadurch gekennzeichnet, dass**
die Übertragungsmechanik einen L-förmigen, doppelt wirkenden Hebel (73) aufweist, dessen Drehpunkt (74) die Hebelarme im Verhältnis von mindestens 2:1 teilt.

28. Verstärkungsimplantat nach Anspruch 26 oder 27,
**dadurch gekennzeichnet, dass**
die Übertragungsmechanik im Schaft (70) angeordnet ist.

29. Verstärkungsimplantat nach einem der Ansprüche 26 bis 28,
**dadurch gekennzeichnet, dass**
die Übertragungsmechanik so ausgebildet ist, dass die Spreizer eine Linearbewegung ausführen.

30. Verstärkungsimplantat nach einem der Ansprüche 12 bis 14,
**dadurch gekennzeichnet, dass**
ein Antriebsinstrument (80) vorgesehen ist, welches einen Schaft (81) mit einem Kupplungskopf an einem und einem Handgriff (84) am anderen Ende sowie eine zur Seite weisende Winkelmarkierung umfasst.

31. Verstärkungsimplantat nach Anspruch 30,
**dadurch gekennzeichnet, dass**
der Hangriff (84) und die Winkelmarke miteinander kombiniert sind.

## Claims

1. Reinforcing implant for the lamina (93) of a vertebra (9), comprising a transverse support and one anchoring device for the right-hand side and one anchoring device for the left-hand side of the lamina of the vertebra, wherein an expansion element (1) with a guide device (2) and with cheek elements (4, 4') is provided on the transverse support, which cheek elements (4, 4') are mounted so as to be longitudinally movable along the guide device (2), wherein bearing surfaces (43) for the lamina (93) are formed on outer faces of the cheek elements (4, 4') directed away from each other, and a return stop for the cheek elements (4, 4') is provided,
**characterized in that**
at least one of the cheek elements (4, 4') is provided with an aligning device for modifying the orientation of its bearing surface (43) relative to the guide device (2).

2. Reinforcing implant according to Claim 1, **characterized in that** the return stop is designed as a clamping device (5, 5') acting between cheek elements (4, 4') and guide device (2).

3. Reinforcing implant according to Claim 2, **characterized in that** the return stop has locking elements, which act between cheek elements (4, 4') and guide device (2).

4. Reinforcing implant according to Claim 3, **characterized in that** the locking elements comprise a ribbing and at least one catch engaging therein.

5. Reinforcing implant according to one of Claims 1 to 4, **characterized in that** a clamping screw (50) is provided, which functions as a bearing for the aligning device.

6. Reinforcing implant according to one of Claims 1 to 5, **characterized in that** the second cheek element (4') is provided with an aligning device (5').

7. Reinforcing implant according to one of the preceding claims, **characterized in that** the expansion element (1) has at least one area protruding laterally beyond the cheek elements (4, 4').

8. Reinforcing implant according to one of the preceding claims, **characterized in that** the guide device (2) has a non-circular bar (2"), with a recess of complementary shape in sliding carriages (3") of the cheek elements (4, 4').

9. Reinforcing implant according to Claim 8, **characterized in that** the bar (2") is V-shaped and preferably forms a V angle of 10° to 30°.

10. Reinforcing implant according to one of the preceding claims, **characterized in that** the guide device comprises a fork-shaped rail (2) and a slide (3) guided in the fork interspace.

11. Reinforcing implant according to Claim 10, **characterized in that** the slide (3) is guided on the rail (2) via a groove-and-ridge arrangement (28, 38), with the ridge engaging with a form fit in the groove.

12. Reinforcing implant according to Claim 10 or 11, **characterized in that** a drive device (8), which bears on the rail (2), is arranged on the slide (3).

13. Reinforcing implant according to Claim 12, **characterized in that** the drive device (8) comprises a toothed rack or a screw spindle.

14. Reinforcing implant according to one of Claims 12 and 13, **characterized in that** the drive device (8) is self-locking.

15. Reinforcing implant according to one of the preceding claims, **characterized in that** the bearing surface (43) has pointed protuberances (46).

16. Reinforcing implant according to one of the preceding claims, **characterized in that** the bearing surface (43) is provided with a coating that promotes bone growth.

17. Reinforcing implant according to one of the preceding claims, **characterized in that** the bearing surfaces on the cheek elements (4, 4') are flush with one another.

18. Reinforcing implant according to one of the preceding claims, **characterized in that** at least one fixing tongue (37) is arranged on the cheek elements (4, 4').

19. Reinforcing implant according to Claim 18, **characterized in that** the fixing tongue (37) has a receiving opening (371) for a fastening element.

20. Reinforcing implant according to Claim 19, **characterized in that** the receiving opening (371) is designed for the polyaxial mounting of a pars screw (377).

21. Reinforcing implant according to Claim 19 or 20, **characterized in that** the receiving opening (371) is oblong and is divided by several lugs (372) into a plurality of unambiguous receiving positions for the fastening element.

22. Reinforcing implant according to one of the preceding claims, **characterized in that** receiving couplings for a gripping tool (7) are provided on the expansion element (1).

23. Reinforcing implant according to Claim 22, **characterized in that** the receiving couplings are designed as locating holes (29, 39).

24. Reinforcing implant according to Claim 23, **characterized in that** a spreading instrument (7) is provided, of which the spreading arms are provided with complementary carriers (79) for the receiving coupling.

25. Reinforcing implant according to Claim 24, **characterized in that** the spreading instrument (7) is a spreading forceps comprising a shaft (70) and a handle (71).

26. Reinforcing implant according to Claim 25, **characterized in that** a transmission mechanism is provided that increases the course of an actuating movement acting on the handle (71).

27. Reinforcing implant according to Claim 26, **characterized in that** the transmission mechanism has an L-shaped, double-action lever (73), of which the pivot point (74) divides the lever arms in the ratio of at least 2:1.

28. Reinforcing implant according to Claim 26 or 27, **characterized in that** the transmission mechanism is arranged in the shaft (70).

29. Reinforcing implant according to one of Claims 26 to 28, **characterized in that** the transmission mechanism is designed such that the spreaders execute a linear movement.

30. Reinforcing implant according to one of Claims 12 to 14, **characterized in that** a drive instrument (80) is provided, which comprises a shaft (81) with a coupling head at one end and with a handle (84) at the other end, and also an angle marking facing the side.

31. Reinforcing implant according to Claim 30, **characterized in that** the handle (84) and the angle marking are combined with each other.

## Revendications

1. Implant de renfort pour la lamina (93) d'une vertèbre (9), comprenant un support transversal et un dispositif d'ancrage respectif sur le côté droit et sur le côté gauche de la lamina de la vertèbre, un élément expansible (1) doté d'un dispositif de guidage (2) et d'éléments de mâchoire (4, 4') étant prévu sur le support transversal, les éléments de mâchoire étant montés à coulissement longitudinal le long du dispositif de guidage (2), des surfaces de pose (43) étant formées pour la lamina (93) sur des côtés extérieurs, s'éloignant l'un de l'autre, des éléments de mâchoire (4, 4') et un blocage anti-recul des éléments de mâchoire (4, 4') étant prévu,
**caractérisé en ce que**
au moins l'un des éléments de mâchoire (4, 4') est doté d'un dispositif d'orientation permettant d'orienter de manière modifiable sa surface de pose (43) par rapport au dispositif de guidage (2).

2. Implant de renfort selon la revendication 1, **caractérisé en ce que** le blocage anti-recul est configuré comme dispositif de serrage (5, 5') agissant entre les éléments de mâchoire (4, 4') et le dispositif de guidage (2).

3. Implant de renfort selon la revendication 2, **caractérisé en ce que** le blocage anti-retour présente des éléments d'encliquetage qui agissent entre les éléments de mâchoire (4, 4') et le dispositif de guidage (2).

4. Implant de renfort selon la revendication 3, **caractérisé en ce que** les éléments d'encliquetage comprennent un moletage et au moins un bec d'encliquetage dans lequel ils s'engagent.

5. Implant de renfort selon l'une des revendications 1 à 4, **caractérisé en ce qu'**une vis de serrage (50) qui joue le rôle d'un support pour le dispositif d'orientation est prévue.

6. Implant de renfort selon l'une des revendications 1 à 5, **caractérisé en ce que** le deuxième élément de mâchoire (4') et doté d'un dispositif d'orientation (5').

7. Implant de renfort selon l'une des revendications précédentes, **caractérisé en ce que** l'élément expansible (1) présente au moins une partie qui déborde latéralement au-dessus des éléments de mâchoire (4, 4').

8. Implant de renfort selon l'une des revendications précédentes, **caractérisé en ce que** le dispositif de guidage (2) comporte une tringle (2") non circulaire dotée d'une découpe de forme complémentaire dans des coulisseaux (3") des éléments de mâchoire (4, 4').

9. Implant de renfort selon la revendication 8, **caractérisé en ce que** la tringle (2") est en forme de V et forme de préférence un V d'un angle de 10° à 30°.

10. Implant de renfort selon l'une des revendications précédentes, **caractérisé en ce que** le dispositif de guidage comporte un rail (2) en forme de fourche et un coulisseau (3) guidé dans l'espace intermédiaire entre les dents de la fourche.

11. Implant de renfort selon la revendication 10, **caractérisé en ce que** le coulisseau (3) est guidé par des rainures et languettes (28, 38) dans le rail (2) de telle sorte que la languette s'engage en correspondance géométrique dans la rainure.

12. Implant de renfort selon les revendications 10 ou 11, **caractérisé en ce qu'**un dispositif d'entraînement (8) qui s'appuie sur le rail (2) est disposé sur le coulisseau (3).

13. Implant de renfort selon la revendication 12, **caractérisé en ce que** le dispositif d'entraînement (8) comporte une crémaillère ou une broche filetée.

14. Implant de renfort selon l'une des revendications 12 à 13, **caractérisé en ce que** le dispositif d'entraînement (8) est autobloquant.

15. Implant de renfort selon l'une des revendications précédentes, **caractérisé en ce que** la surface de pose (43) présente des reliefs (46) en pointe.

16. Implant de renfort selon l'une des revendications précédentes, **caractérisé en ce que** la surface de pose (43) est dotée d'un revêtement favorisant la croissance osseuse.

17. Implant de renfort selon l'une des revendications précédentes, **caractérisé en ce que** les surfaces de pose sont alignées l'une sur l'autre sur les éléments de mâchoire (4, 4').

18. Implant de renfort selon l'une des revendications précédentes, **caractérisé en ce qu'**au moins une languette de fixation (37) est disposée sur les éléments de mâchoire (4, 4').

19. Implant de renfort selon la revendication 18, **caractérisé en ce que** la languette de fixation (37) présente une ouverture de réception (371) pour un élément de fixation.

20. Implant de renfort selon la revendication 19, **caractérisé en ce que** l'ouverture de réception (371) est configurée pour un montage polyaxial d'une vis de Pars (377).

21. Implant de renfort selon les revendications 19 ou 20, **caractérisé en ce que** l'ouverture de réception (371) est allongée et divisée par plusieurs becs (372) en plusieurs positions nettes de réception de l'élément de fixation.

22. Implant de renfort selon l'une des revendications précédentes, **caractérisé en ce que** des accouplements de réception d'un outil de serrage (7) sont prévus sur l'élément expansible (1).

23. Implant de renfort selon la revendication 22, **caractérisé en ce que** les accouplements de réception sont configurés comme trous de passage (29, 39).

24. Implant de renfort selon la revendication 23, **caractérisé en ce qu'**un instrument écarteur (7) est prévu sur les bras écarteur duquel sont prévus des dispositifs d'entraînement (79) complémentaires pour l'accouplement de réception.

25. Implant de renfort selon la revendication 24, **caractérisé en ce que** l'instrument d'écartement (7) est une pince d'écartement qui comporte une tige (70) et une poignée (71).

26. Implant de renfort selon la revendication 25, **caractérisé en ce qu'**une mécanique de transfert qui augmente le parcours d'actionnement agissant sur la poignée (71) est prévue.

27. Implant de renfort selon la revendication 26, **caractérisé en ce que** la mécanique de transfert présente un levier (73) en forme de L et à double action dont le centre de rotation (74) divise les bras du levier dans un rapport d'au moins 2:1.

28. Implant de renfort selon les revendications 26 ou 27, **caractérisé en ce que** la mécanique de transfert est disposée dans la tige (70).

29. Implant de renfort selon l'une des revendications 26 à 28, **caractérisé en ce que** la mécanique de transfert est configurée de telle sorte que les écarteurs exécutent un déplacement linéaire.

30. Implant de renfort selon l'une des revendications 12 à 14, **caractérisé en ce qu'**il présente un instrument d'entraînement (80) qui comporte une tige (81) dotée d'une tête d'accouplement sur une extrémité et une poignée (84) à l'autre extrémité, ainsi qu'un repère angulaire orienté vers le côté.

31. Implant de renfort selon la revendication 30, **caractérisé en ce que** la poignée (84) et les repères angulaires sont combinés mutuellement.
